# EUROPEAN PATENT APPLICATION

(11) **EP 3 034 618 A1**
(43) Date of publication of application: **22.06.2016**
(21) Application number: 14198226.4
(22) Date of filing: 16.12.2014
(51) Int. Cl.: C12Q 1/68

(54) **A nucleic acid CMOS based chip for diagnosing periodontal infectious disease**

(71) Applicant: Isamat Riviere, Marcos, 08022 Barcelona (ES)
(72) Inventor: Isamat Riviere, Marcos, 08022 Barcelona (ES)
(74) Representative: Balder IP Law, S.L.

(57) **Abstract**

A nucleic acid CMOS based chip for diagnosing periodontal infectious disease, which comprises a probe capable of hybridizing with a fragment containing the 18S-RNA gene (Accession number: gi|387119833:446-659) of the bacterium *Aggregatibacter actinomycetecomitans* and a CMOS detector chip comprising a specific combination of at least fourteen periodontal pathogens genome fragments as a target.

## Description

### TECHNICAL FIELD

CMOS chips for detecting pathogens in human oral cavities.

### STATE OF THE ART

It is known in the art to use CMOS chips for detecting pathogens. The main aspects of this technique are disclosed in the following patent documents form InSilixa, US 8,637,436 B2, US 8,048,626 B2, US 11/376,398, US/758,621, EP07784330.8, US 09/514502, US 11/829,861, US 13/240,603, US 11/844,996, US 12/617,794, US 13/417,661, US/959,492, US 13/535,665, US 61/049,204, US 12/433,896, US 12/507,142, which contents are to be considered as a part of the present description.

The present inventors have found that up to now, no combination of probes that avoid cross-reactivity and that provide for specific detection are available for detecting pathogens in the oral cavity.

### DESCRIPTION OF THE INVENTION

To satisfy this need, the present invention provides for a:

A nucleic acid CMOS based chip for diagnosing periodontal infectious disease, which comprises a probe capable of hybridizing with a fragment containing the 18S-RNA gene (Accession number: gi|387119833:446-659) of the bacterium *Aggregatibacter actinomycetecomitans.*

A nucleic acid CMOS based chip for diagnosing periodontal infectious disease according to claim 1, which comprises a probe capable of hybridizing with a fragment containing the 18S-RNA gene (Accession number: gi|174016|gb|L04317.1|CAJRRDAF:58-238) of the bacterium *Campylobacter rectus.*

A nucleic acid CMOS based chip for diagnosing periodontal infectious disease according to any of the previous claims, which comprises a probe capable of hybridizing with a fragment containing the 18S-RNA gene (Accession number: gi|334145811:1052-1246) of the bacterium *Porphyromonas gingivalis.*

A nucleic acid CMOS based chip for diagnosing periodontal infectious disease according to any of the previous claims, which comprises a probe capable of hybridizing with a fragment containing the 18S-RNA gene (Accession number: gi|444304158|ref|NR_074582.1|:438-627) of the bacterium *Treponema denticola.*

A nucleic acid CMOS based chip for diagnosing periodontal infectious disease according to any of the previous claims, which comprises a probe capable of hybridizing with a fragment containing the DNApol-A gene (Accession number: gb|ACLQ01000023.1|:1402-1621) of the bacterium *Capnocytophaga gingivalis.*

A nucleic acid CMOS based chip for diagnosing periodontal infectious disease according to any of the previous claims, which comprises a probe capable of hybridizing with a fragment containing the DNApol-A gene (Accession number: gb|CP001632.1|:954-1170) of the bacterium *Capnocytophaga ochracea.*

A nucleic acid CMOS based chip for diagnosing periodontal infectious disease according to any of the previous claims, which comprises a probe capable of hybridizing with a fragment containing the DNApol-A gene (Accession number: gi|334145811:527-728) of the bacterium *Prevotella intermedia.*

A nucleic acid CMOS based chip for diagnosing periodontal infectious disease according to any of the previous claims, which comprises a probe capable of hybridizing with a fragment containing the DNApol-A gene (Accession number: gi|387131618:1422-1624) of the bacterium *Parvimonas micra.*

A nucleic acid CMOS based chip for diagnosing periodontal infectious disease according to any of the previous claims, which comprises a probe capable of hybridizing with a fragment containing the DNApol-A gene (Accession number: gi|375253814:306-494) of the bacterium *Tannerella forsythia.*

A nucleic acid CMOS based chip for diagnosing periodontal infectious disease according to any of the previous claims, which comprises a probe capable of hybridizing with a fragment containing the DNApol-A gene (Accession number: gb|GG665898.1|:421-614) of the bacterium *Fusobacterium periodonticum*

A nucleic acid CMOS based chip for diagnosing periodontal infectious disease according to any of the previous claims, which comprises a probe capable of hybridizing with a fragment containing the DNApol-A gene (Accession number: gb|ABZV01000011.1|:2319-2516) of the bacterium *Capnocytophaga sputigena*

A nucleic acid CMOS based chip for diagnosing periodontal infectious disease according to any of the previous claims, which comprises a probe capable of hybridizing with a fragment containing the DNApol-A gene (Accession number: gb|EQ973317.1|:1761-1947) of the bacterium *Eikenella corrodens*

A nucleic acid CMOS based chip for diagnosing periodontal infectious disease according to any of the previous claims, which comprises a probe capable of hybridizing with a fragment containing the DNApol-A gene (Accession number: gi|19703352:1932-2142) of the bacterium *Fusobacterium nucleatum*

A nucleic acid CMOS based chip for diagnosing periodontal infectious disease according to any of the previous claims, which comprises a probe capable of hybridizing with a fragment containing the DNApol-A gene (Accession number: gi|544921415:2299-2494) of the bacterium *Treponema socanskrii*

Finally, the present invention provides for a CMOS detector chip comprising a specific combination of at least fourteen periodontal pathogens genome fragments as a target, said combination including the following genetic markers to identify species:

| **Target (File Name) and accession number** | **Primer Name** | **Sequence** |
|---|---|---|
| 18S-TDM-Aactinomycetecomitans gi\|387119833:446-659 | Aactm-F | GGTTGGTGTGTTAATAGCATGCCA |
| | Aactm-R | CCCTAAAGTACTCCAGACCCCC |
| 18S-TDM-Crectus gi\|74016\|gb\|L04317.1\|CAJRRDAF:5 8-238 | Crect-F | CAAGTCGAACGGAGATTAAGTAGC T |
| | Crect-R | ACGATATAGCCTCATCCTACACCG |
| 18S-TDM-Pgingivalis gi\|334145811:1052-1246 | Pgingiv-F | TGCAATACTCGTATCGCCCGTTA |
| | Pgingiv-R | ACGATGGGTAGGGGAACTGAGA |
| 18S-TDM-Tdenticola gi\|444304158\|ref\|NR_074582.1\|:438-627 | Tdent-F | GGGAATGCTTCTTTGATGACGGTA |
| | Tdent-R | AGTCAAGCAGTACCCAATGCAGT |
| DNApolA-TDM-Cgingivalis gb\|ACLQ01000023.1\|:1402-1621 | Cgingic-F | AGCTATGCCCCTGTTGCCAT |
| | Cgingic-R | TCTGAGCCAATACGCGAACCA |
| DNApolA-TDM-Cochracea gb\|CP001632.1\|:954-1170 | Cochra-F | CGACCTATTTGCCCAACCGC |
| | Cochra-R | ACCTTTGTGAGCCGACCAGC |
| DNApolA-TDM-Pintermedia gi\|334145811:527-728 | Pint-F | GTTTCCGACCGCCCTACATTG |
| | Pint-R | GCGATTTTCTGGTTGGCAGATGT |
| DNApolA-TDM-Pmicra gi\|387131618:1422-1624 | Pmicra-F | TCAAAGCAATCCTCTCTCCAAAAC T |
| | Pmicra-R | ATTACCAGAAATGAAGCAAGGCAG T |
| DNApolA-TDM-Tforsythia gi\|375253814:306-494 | Tfors-F | GGATGTCGGGGAGGAAACGT |
| | Tfors-R | ACGGCATCTCGGTGTTTGGA |
| DNApolA-TDM-Fperiodonticum gb\|GG665898.1\|:421-614 | Fperio-F | GCTCTTCGTCGTGGCAAGAG |
| | Fperio-R | TCCACAGTCTTTCACCTGATCCTT |
| DNApolA-TDM-Csputigena gb\|ABZV01000011.1\|:2319-2516 | Csput-F | ATCCGCACAAACCGACCTCT |
| | Csput-R | GCACCTCCACGCACTACTTG |
| DNApolA-TDM-Ecorrodens gb\|EQ973317.1\|:1761-1947 | Ecorred-F | CGCTTGCCTGTTTTGCCTCA |
| | Ecorred-R | CCGGAAGCAGGTGGGGTATT |
| DNApolA-TDM-Fnucleatum gi\|19703352:1932-2142 | Fnucl-F | AGGAACAGCAACAGGGAGACT |
| | Fnucl-R | ATCCTTTTCTTCCCTGTATGCTTCT |
| DNApolA-TDM-Tsocanskrii gi\|544921415:2299-2494 | Tsoc-F | AACGCCGTTGGAATCGATCG |
| | Tsoc-R | CATTCCGATCCTGCAGTGCG |

This no-evident selection allows to detect the main periodontal pathogens in a simple and fast way and it avoids cross-reactivity.

Specifically, all sequences selected form the 14 toxic periodontal pathogens have been searched for specifity and lack of cross-reactivity from a pool of microorganisms genomes normally present as normal components of the biota (microbial flora) of the human oral cavity.

The human oral cavity is known to normally have microbial flora that does not contain pathological bacterial flora as part of the normal physiological state of the oral cavity.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:

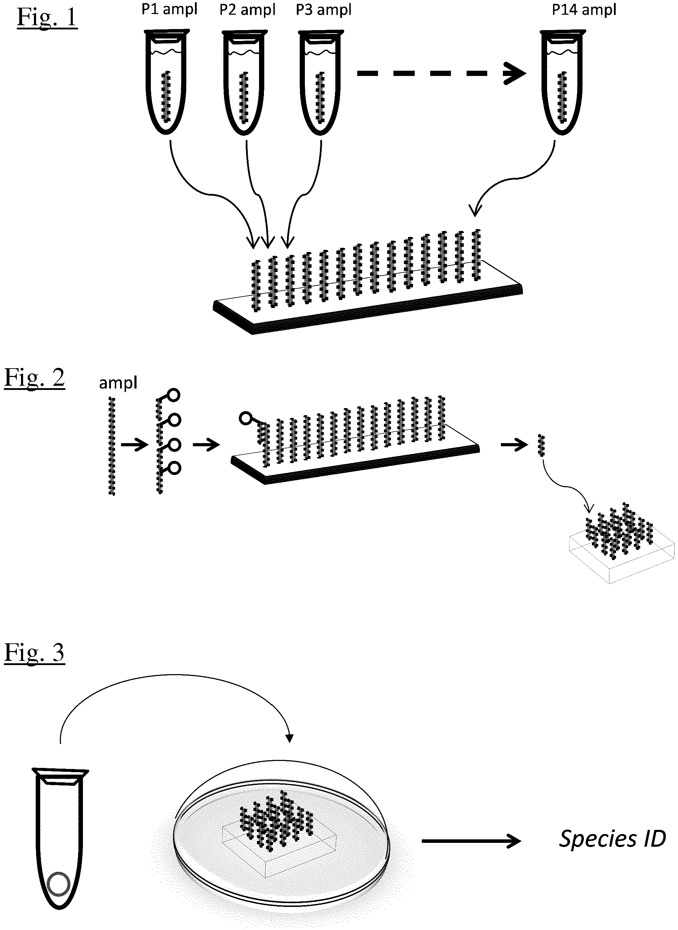

Figure 1 to 3 show the three main steps employed for the detection of the pathogens using the CMOS based chip of the present invention.

### DESCRIPTION OF A WAY OF CARRYING OUT THE INVENTION

In FIG. 1 a conventional PCR is performed separately for each of the 14 periodontal pathogen genomes using the primer pair stated on primer table in the text (Aactm-F and Aactm-R; Crect-F and Crect-R; Pgingiv-F and Pgingiv-R; Tdent-F and Tdent-R; Cgingic-F and Cgingic-R; Cochra-F and Cochra-R; Pint-F and Pint-R; Pmicra-F and Pmicra-R; Tfors-F and Tfors-R; Fperio-F and Fperio-R; Csput-F and Csput-R; Ecorred-F and Ecorred-R; Fnucl-F and Fnucl-R; Tsoc-F and Tsoc-R). The amplicons resulting from the 14 PCR reactions are spotted on a glass slide to test for optimal probes aimed at CMOS chip inclusion.

In FIG. 2 an optimal probe selection for eventual printing on CMOS-chip is performed by hybridization of 20 nucleotide long synthetic oligonucleotide probes spanning each of the DNA sequences used in (a). Probes at this step are labelled with a fluorescent molecule and hybridized conventionally, both in separate and in multiple combinations with probes from all 14 pathogen species, to the printed glass slide obtained in (a). Results are read with a densitometer and probe selection for the CMOS-chip functionalization is based on specificity, sensitivity and cross reactivity measurements.

In FIG. 3 probes selected in this way are printed on the CMOS-chip in triplicate for each of the 14 periodontal pathogens. A biological sample of saliva is added to the reaction chamber of the CMOS-chip containing all PCR reagents including all primers as stated in (a), presence of a specific pathogen in the saliva sample is revealed by hybridization on the corresponding probe attached to a known pixel on the CMOS-chip surface.

The invention is obviously not limited to the specific embodiment described herein, but also encompasses any variations that may be considered by any person skilled in the art within the general scope of the invention as defined in the claims.

| **Target (File Name) and accession number** | **Primer Name** | **Sequence** |
|---|---|---|
| 18S-TDM-Aactinomycetecomitans gi\|387119833:446-659 | Aactm-F | GGTTGGTGTGTTAATAGCATGCCA |
| | Aactm-R | CCCTAAAGTACTCCAGACCCCC |
| 18S-TDM-Crectus gi\|174016\|gb\|L04317.1\|CAJRRDAF:58-238 | Crect-F | CAAGTCGAACGGAGATTAAGTAGCT |
| | Crect-R | ACGATATAGCCTCATCCTACACCG |
| 18S-TDM-Pgingivalis gi\|334145811:1052-1246 | Pgingiv-F | TGCAATACTCGTATCGCCCGTTA |
| | Pgingiv-R | ACGATGGGTAGGGGAACTGAGA |
| 18S-TDM-Tdenticola gi\|444304158\|ref\|NR_074582.1\|:438-627 | Tdent-F | GGGAATGCTTCTTTGATGACGGTA |
| | Tdent-R | AGTCAAGCAGTACCCAATGCAGT |
| DNApolA-TDM-Cgingivalis gb\|ACLQ01000023.1\|:1402-1621 | Cgingic-F | AGCTATGCCCCTGTTGCCAT |
| | Cgingic-R | TCTGAGCCAATACGCGAACCA |
| DNApolA-TDM-Cochracea gb\|CP001632.1\|:954-1170 | Cochra-F | CGACCTATTTGCCCAACCGC |
| | Cochra-R | ACCTTTGTGAGCCGACCAGC |
| DNApolA-TDM-Pintermedia gi\|334145811:527-728 | Pint-F | GTTTCCGACCGCCCTACATTG |
| | Pint-R | GCGATTTTCTGGTTGGCAGATGT |
| DNApolA-TDM-Pmicra gi\|387131618:1422-1624 | Pmicra-F | TCAAAGCAATCCTCTCTCCAAAACT |
| | Pmicra-R | ATTACCAGAAATGAAGCAAGGCAGT |
| DNApolA-TDM-Tforsythia gi\|375253814:306-494 | Tfors-F | GGATGTCGGGGAGGAAACGT |
| | Tfors-R | ACGGCATCTCGGTGTTTGGA |
| DNApolA-TDM-Fperiodonticum gb\|GG665898.1\|:421-614 | Fperio-F | GCTCTTCGTCGTGGCAAGAG |
| | Fperio-R | TCCACAGTCTTTCACCTGATCCTT |
| DNApolA-TDM-Csputigena gb\|ABZV01000011.1\|:2319-2516 | Csput-F | ATCCGCACAAACCGACCTCT |
| | Csput-R | GCACCTCCACGCACTACTTG |
| DNApolA-TDM-Ecorrodens gb\|EQ973317.1\|:1761-1947 | Ecorred-F | CGCTTGCCTGTTTTGCCTCA |
| | Ecorred-R | CCGGAAGCAGGTGGGGTATT |
| DNApolA-TDM-Fnucleatum gi\|19703352:1932-2142 | Fnucl-F | AGGAACAGCAACAGGGAGACT |
| | Fnucl-R | ATCCTTTTCTTCCCTGTATGCTTCT |
| DNApolA-TDM-Tsocanskrii gi\|544921415:2299-2494 | Tsoc-F | AACGCCGTTGGAATCGATCG |
| | Tsoc-R | CATTCCGATCCTGCAGTGCG |

## Claims

1. A nucleic acid CMOS based chip for diagnosing periodontal infectious disease, which comprises a probe capable of hybridizing with a fragment containing the 18S-RNA gene (Accession number: gi|387119833:446-659) of the bacterium *Aggregatibacter actinomycetecomitans.*

2. A nucleic acid CMOS based chip for diagnosing periodontal infectious disease according to claim 1, which comprises a probe capable of hybridizing with a fragment containing the 18S-RNA gene (Accession number: gi|174016|gb|L04317.1|CAJRRDAF:58-238) of the bacterium *Campylobacter rectus.*

3. A nucleic acid CMOS based chip for diagnosing periodontal infectious disease according to any of the previous claims, which comprises a probe capable of hybridizing with a fragment containing the 18S-RNA gene (Accession number: gi|334145811:1052-1246) of the bacterium *Porphyromonas gingivalis.*

4. A nucleic acid CMOS based chip for diagnosing periodontal infectious disease according to any of the previous claims, which comprises a probe capable of hybridizing with a fragment containing the 18S-RNA gene (Accession number: gi|444304158|ref|NR_074582.1|:438-627) of the bacterium *Treponema denticola.*

5. A nucleic acid CMOS based chip for diagnosing periodontal infectious disease according to any of the previous claims, which comprises a probe capable of hybridizing with a fragment containing the DNApol-A gene (Accession number: gb|ACLQ01000023.1|:1402-1621) of the bacterium *Capnocytophaga gingivalis.*

6. A nucleic acid CMOS based chip for diagnosing periodontal infectious disease according to any of the previous claims, which comprises a probe capable of hybridizing with a fragment containing the DNApol-A gene (Accession number: gb|CP001632.1|:954-1170) of the bacterium *Capnocytophaga ochracea.*

7. A nucleic acid CMOS based chip for diagnosing periodontal infectious disease according to any of the previous claims, which comprises a probe capable of hybridizing with a fragment containing the DNApol-A gene (Accession number: gi|334145811:527-728) of the bacterium *Prevotella intermedia.*

8. A nucleic acid CMOS based chip for diagnosing periodontal infectious disease according to any of the previous claims, which comprises a probe capable of hybridizing with a fragment containing the DNApol-A gene (Accession number: gi|387131618:1422-1624) of the bacterium *Parvimonas micra.*

9. A nucleic acid CMOS based chip for diagnosing periodontal infectious disease according to any of the previous claims, which comprises a probe capable of hybridizing with a fragment containing the DNApol-A gene (Accession number: gi|375253814:306-494) of the bacterium *Tannerella forsythia.*

10. A nucleic acid CMOS based chip for diagnosing periodontal infectious disease according to any of the previous claims, which comprises a probe capable of hybridizing with a fragment containing the DNApol-A gene (Accession number: gb|GG665898.1|:421-614) of the bacterium *Fusobacterium periodonticum*

11. A nucleic acid CMOS based chip for diagnosing periodontal infectious disease according to any of the previous claims, which comprises a probe capable of hybridizing with a fragment containing the DNApol-A gene (Accession number: gb|ABZV01000011.1|:2319-2516) of the bacterium *Capnocytophaga sputigena*

12. A nucleic acid CMOS based chip for diagnosing periodontal infectious disease according to any of the previous claims, which comprises a probe capable of hybridizing with a fragment containing the DNApol-A gene (Accession number: gb|EQ973317.1|:1761-1947) of the bacterium *Eikenella corrodens*

13. A nucleic acid CMOS based chip for diagnosing periodontal infectious disease according to any of the previous claims, which comprises a probe capable of hybridizing with a fragment containing the DNApol-A gene (Accession number: gi|19703352:1932-2142) of the bacterium *Fusobacterium nucleatum*

14. A nucleic acid CMOS based chip for diagnosing periodontal infectious disease according to any of the previous claims, which comprises a probe capable of hybridizing with a fragment containing the DNApol-A gene (Accession number: gi|544921415:2299-2494) of the bacterium *Treponema socanskrii*

15. CMOS detector chip comprising a specific combination of at least fourteen periodontal pathogens genome fragments as a target, said combination including the following genetic markers to identify species:
| **Target (File Name) and accession number** | **Primer Name** | **Sequence** |
|---|---|---|
| 18S-TDM-Aactinomycetecomitans gi\|387119833:446-659 | Aactm-F | GGTTGGTGTGTTAATAGCATGCCA |
| | Aactm-R | CCCTAAAGTACTCCAGACCCCC |
| 18S-TDM-Crectus gi\|174016\|gb\|L04317.1\|CAJRRDAF:5 8-238 | Crect-F | CAAGTCGAACGGAGATTAAGTAGC T |
| | Crect-R | ACGATATAGCCTCATCCTACACCG |
| 18S-TDM-Pgingivalis gi\|334145811:1052-1246 | Pgingiv-F | TGCAATACTCGTATCGCCCGTTA |
| | Pgingiv-R | ACGATGGGTAGGGGAACTGAGA |
| 18S-TDM-Tdenticola gi\|444304158\|ref\|NR_074582.1\|:438-627 | Tdent-F | GGGAATGCTTCTTTGATGACGGTA |
| | Tdent-R | AGTCAAGCAGTACCCAATGCAGT |
| DNApolA-TDM-Cgingivalis gb\|ACLQ01000023.1\|:1402-1621 | Cgingic-F | AGCTATGCCCCTGTTGCCAT |
| | Cgingic-R | TCTGAGCCAATACGCGAACCA |
| DNApolA-TDM-Cochracea gb\|CP001632.1\|:954-1170 | Cochra-F | CGACCTATTTGCCCAACCGC |
| | Cochra-R | ACCTTTGTGAGCCGACCAGC |
| DNApolA-TDM-Pintermedia gi\|334145811:527-728 | Pint-F | GTTTCCGACCGCCCTACATTG |
| | Pint-R | GCGATTTTCTGGTTGGCAGATGT |
| DNApolA-TDM-Pmicra gi\|387131618:1422-1624 | Pmicra-F | TCAAAGCAATCCTCTCTCCAAAAC T |
| | Pmicra-R | ATTACCAGAAATGAAGCAAGGCAG T |
| DNApolA-TDM-Tforsythia gi\|375253814:306-494 | Tfors-F | GGATGTCGGGGAGGAAACGT |
| | Tfors-R | ACGGCATCTCGGTGTTTGGA |
| DNApolA-TDM-Fperiodonticum gb\|GG665898.1\|:421-614 | Fperio-F | GCTCTTCGTCGTGGCAAGAG |
| | Fperio-R | TCCACAGTCTTTCACCTGATCCTT |
| DNApolA-TDM-Csputigena gb\|ABZV01000011.1\|:2319-2516 | Csput-F | ATCCGCACAAACCGACCTCT |
| | Csput-R | GCACCTCCACGCACTACTTG |
| DNApolA-TDM-Ecorrodens gb\|EQ973317.1\|:1761-1947 | Ecorred-F | CGCTTGCCTGTTTTGCCTCA |
| | Ecorred-R | CCGGAAGCAGGTGGGGTATT |
| DNApolA-TDM-Fnucleatum gi\|19703352:1932-2142 | Fnucl-F | AGGAACAGCAACAGGGAGACT |
| | Fnucl-R | ATCCTTTTCTTCCCTGTATGCTTCT |
| DNApolA-TDM-Tsocanskrii gi\|544921415:2299-2494 | Tsoc-F | AACGCCGTTGGAATCGATCG |
| | Tsoc-R | CATTCCGATCCTGCAGTGCG |
